# NEUE EUROPÄISCHE PATENTSCHRIFT

(11) **EP 0 929 647 B2**
(45) Veröffentlichungstag und Bekanntmachung des Hinweises auf die Entscheidung über den Einspruch: **29.03.2006**
(45) Hinweis auf die Patenterteilung: 29.11.2000
(21) Anmeldenummer: 97910389.2
(22) Anmeldetag: 29.09.1997
(51) Int. Cl.: C11D 11/00, C11D 1/28, C11D 1/83, C11D 1/86, C11D 17/06

(54) **VERFAHREN ZUR HERSTELLUNG WASSER- UND STAUBFREIER ANIONTENSIDGRANULATE**
METHOD FOR THE PRODUCTION OF WATERFREE AND DUSTFREE ANIONIC SURFACTANTS
PROCEDE DE PRODUCTION DE GRANULES DE TENSIOACTIFS ANIONIQUES ANHYDRES ET SANS POUSSIERE

(30) Priorität: 07.10.1996 DE 19641275
(43) Veröffentlichungstag der Anmeldung: 21.07.1999
(73) Patentinhaber: Cognis IP Management GmbH, 40589 Düsseldorf (DE)
(72) Erfinder: LÜDER, Thomas, D-40764 Langenfeld (DE); FABRY, Bernd, D-41352 Korschenbroich (DE); KAHRE, Jörg, D-42799 Leichlingen (DE); SEIPEL, Werner, D-40723 Hilden (DE)
(86) Internationale Anmeldenummer: PCT/EP1997/005348
(87) Internationale Veröffentlichungsnummer: WO 1998/015611

(56) Entgegenhaltungen:
- EP-A- 0 384 480
- EP-A- 0 572 957
- WO-A-93/19155
- WO-A-96/06916
- DE-C- 19 534 371
- US-A- 5 536 431
- DATABASE WPI Section Ch, Week 9636 Derwent Publications Ltd., London, GB; Class D25, AN 96-358656 XP002058428 & JP 08 170 093 A (LION CORP) , 2.Juli 1996

## Beschreibung

### Gebiet der Erfindung

Die Erfindung betrifft ein Verfahren zur gleichzeitigen Trocknung und Granulierung von wäßrigen Monoglycerid(ether)sulfat-Pasten unter Verwendung eines Dünnschichtverdampfers.

### Stand der Technik

Monoglycerid(ether)sulfate zeichnen sich durch ausgezeichnete Detergenseigenschaften und hohe ökotoxikologische Verträglichkeit aus. Aus diesem Grund gewinnen diese anionischen Tenside in zunehmendem Maße an Bedeutung. Werden sie bislang in der Regel in flüssigen Formulierungen, wie beispielsweise Geschirrspülmitteln oder Haarshampoos eingesetzt, so besteht inzwischen auch ein Marktbedürfnis nach festen, wasserfreien Anbietungsformen, die sich beispielsweise auch in Pulverwaschmittel, Zahncremes oder Syndetseifen einarbeiten lassen.

Die Trocknung flüssiger Tensidzubereitungen erfolgt großtechnisch in der Regel durch konventionelle Sprühtrocknung, bei der man die wäßrige Tensidpaste am Kopf eines Turmes in Form feiner Tröpfchen versprüht, denen heiße Trocknungsgase entgegengeführt werden. Die drastischen Bedingungen führen indes häufig zu Hydrolyseerscheinungen, unerwünschten Verfärbungen der Produkte, Anbackungen an den Wandungen der Sprühtürme und in der Folge zu Verunreinigungen des Trockengutes durch verkohlte Rückstände. Die immer wieder erforderliche Reinigung der Türme führt zudem zu Ausfallzeiten, was das Verfahren auch mitunter kostspielig macht. Ein weiteres Problem besteht ferner darin, daß Verfahren des Stands der Technik nicht zu den besonders bevorzugten Schwerpulvern mit einem Schüttgewicht oberhalb von 500 g/l mit einem gleichzeitig stark verminderten Staubgehalt führt. Gerade diese beiden Parameter sind jedoch aus wirtschaftlichen, anwendungstechnischen und sicherheitstechnischen Gründen von besonderer Bedeutung.

Aus der deutschen Offenlegungsschrift **DE-A1 4209339** (Henkel) ist ein Verfahren zur Herstellung rieselfähiger Wasch- und Reinigungsmittelgranulate bekannt, bei dem die Entwässerung in Turbinentrocknern, d.h. in zylindrischen Trockenapparaturen in horizontaler Bauweise mit rotierenden Einbauten erfolgt. Dabei können auch Monoglyceridsulfate enthaltende Reinigungsmittelpasten getrocknet werden. Pulverförmige Monoglyceridsulfate werden des weiteren auch in der **DE-PS 725474** erwähnt.

Demzufolge hat die komplexe Aufgabe der vorliegenden Erfindung darin bestanden, wäßrige Monoglycerid (ether)sulfat-Pasten mit möglichst geringem technischen Aufwand ohne Mitverwendung von anorganischen oder organischen Trägern in praktisch wasser- und staubfreie Granulate zu überführen, die sich gleichzeitig durch eine akzeptable Farbqualität, ein hohes Schüttgewicht, gute Rieselfähigkeit, eine zufriedenstellende Lagerstabilität und im Vergleich zu den Produkten des Stands der Technik durch zumindest vergleichbare anwendungstechnische Eigenschaften auszeichnen.

### Beschreibung der Erfindung

Gegenstand der Erfindung ist ein Verfahren zur Herstellung von wasser- und staubfreien Aniontensidgranulaten mit hohem Schüttgewicht, bei dem man wäßrige Pasten von Monoglycerid(ether)sulfaten mit einem Feststoffgehalt von mindestens 20 und vorzugsweise im Bereich von 25 bis 75 Gew.-% in einem horizontal angeordneten Dünnschichtverdampfer mit rotierenden Einbauten bis auf einen Restwassergehalt unterhalb von 1 Gew.-% gleichzeitig trocknet und in stückige Form bringt, wobei man an den Dünnschichtverdampfer vom Produkteingang bis zum Produktaustrag einen negativen Temperaturgradienten anlegt, im Gegenstrom mit Luft begast und die Trocknung ausschließlich über die beheizte Wandung erfolgt.

Überraschenderweise wurde gefunden, daß ein horizontal angeordneter Dünnschichtverdampfer in idealer Weise geeignet ist, wäßrige Monoglycerid(ether)sulfat-Pasten in trockene, hellfarbige, rieselfähige und nicht-klebrige Granulate zu überführen, ohne daß es zu Produktverfärbungen und Anbacken an den Wandungen kommt. Die Produkte weisen eine hohe Schüttdichte im Bereich von 550 bis 650 g/l und einen mittleren Korndurchmesser von 2,0 bis 2,8 mm auf, was zu einer Verminderung der unerwünschten Wasseraufnahme und des Zusammenbackens der Partikel führt. Auf diese Weise ist auch eine hohe Lagerstabilität gegeben. Gleichzeitig sind sie staubfrei, d.h. der Anteil an Teilchen mit einem Durchmesser unterhalb von 200 pm liegt unterhalb von 5 Gew.-%. Diese Erkenntnis ist um so überraschender, da Bauteile der genannten Art zwar grundsätzlich für die Trocknung von Wertstoffen bekannt sind, die dabei resultierenden Pulver jedoch weder im Hinblick auf das Schüttgewicht, noch den Staub- und Restwassergehalt die genannten Anforderungen erfüllen. In einer besonderen Ausführungsform der Erfindung, werden Mischungen von Monoglycerid(ether)sulfaten und Alkyl- und/oder Alkenyloligoglykosiden und/oder Fettsäure-N-alkylglucamiden eingesetzt.

### Monoglycerid(ether)sulfate

Monoglyceridsulfate und Monoglyceridethersulfate stellen bekannte anionische Tenside dar, die nach den einschlägigen Methoden der präparativen organischen Chemie erhalten werden können. Üblicherweise geht man zu ihrer Herstellung von Triglyceriden aus, die gegebenenfalls nach Ethoxylierung zu den Monoglyceriden umgeestert und nachfolgend sulfatiert und neutralisiert werden. Gleichfalls ist es möglich, die Partialglyceride mit geeigneten Sulfatierungsmitteln, vorzugsweise gasförmiges Schwefeltrioxid oder Chlorsulfonsäure umzusetzen [vgl. **EP-B1 0561825, EP-B1 0561999** (Henkel)]. Die neutralisierten Stoffe können - falls gewünscht - einer Ultrafiltration unterworfen werden, um den Elektrolytgehalt auf ein gewünschtes Maß zu vermindern [**DE-A1 42 04 700** (Henkel)]. Übersichten zur Chemie der Monoglyceridsulfate sind beispielsweise von A.K.Biswas et al. in **J.Am.Oil.Chem.Soc. 37, 171 (1960)** und F.U. Ahmed **J.Am.Oil.Chem.Soc. 67, 8 (1990)** erschienen. Die im Sinne der Erfindung einzusetzenden Monoglycerid (ether)sulfate folgen der Formel **(I)**, 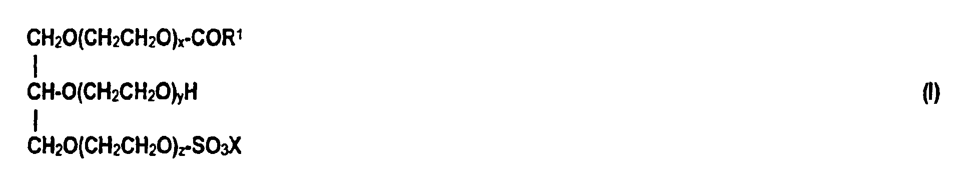 in der R¹CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali-oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (I) eingesetzt, in der R¹ CO für einen linearen Acylrest mit 8 bis 18 Kohlenstoffatomen steht.

### Alkyl- und/oder Alkenyloligoglykoside

Alkyl- und Alkenyloligoglykoside stellen bekannte nichtionische Tenside dar, die der Formel (II) folgen,

R²O-[G]ₚ (II)

in der R² für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften EP-**A1-0301298** und **WO 90/03977** verwiesen. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel (II) gibt den Oligomerisierungsgrad (DP-Grad), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl-bzw. Alkenylrest R² kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R² kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### Fettsäure-N-alkylpolyhydroxyalkylamide

Fettsäure-N-alkylpolyhydroxyalkylamide stellen nichtionische Tenside dar, die der Formel (III) folgen, 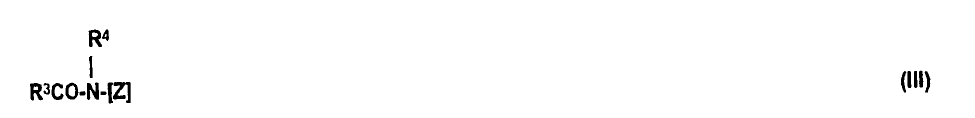 in der R³CO für einen aliphatischen Acylrest mit 6 bis 22 Kohlenstoffatomen, R⁴ für einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Bei den Fettsäure-N-alkylpolyhydroxyalkylamiden handelt es sich um bekannte Stoffe, die üblicherweise durch reduktive Aminierung eines reduzierenden Zuckers mit einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften **US 1,985,424, US 2,016,962** und **US 2,703,798** sowie die Internationale Patentanmeldung **WO 92/06984** verwiesen. Eine Übersicht zu diesem Thema von H.Kelkenberg findet sich in **Tens.Surf.Det. 25, 8 (1988)**. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher **Fettsäure-N-alkylglucamide** dar, wie sie durch die Formel (IV) wiedergegeben werden:

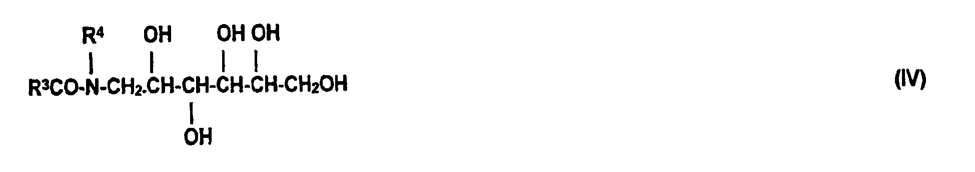

Vorzugsweise werden als Fettsäure-N-alkylpolyhydroxyalkylamide Glucamide der Formel (IV) eingesetzt, in der R⁴ für eine Alkylgruppe steht und R³CO den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen darstellt. Besonders bevorzugt sind Fettsäure-N-alkylglucamide der Formel (IV), die durch reduktive Aminierung von Glucose mit Methylamin und anschließende Acylierung mit Laurinsäure oder C_{12/14}-Kokosfettsäure bzw. einem entsprechenden Derivat erhalten werden. Weiterhin können sich die Polyhydroxyalkylamide auch von Maltose und Palatinose ableiten.

### Trocknen und Granulieren im Flashdryer

Die gleichzeitige Trocknung und Granulierung erfolgt in einem horizontal angeordneten Dünnschichtverdampfer mit rotierenden Einbauten, wie er z.B. von der Firma VRV unter der Bezeichnung "Flashdryer" vertrieben wird. Hierbei handelt es sich, vereinfacht dargestellt, um ein Rohr, das über mehre Zonen hinweg unterschiedlich temperiert werden kann. Über eine oder mehrere Wellen, die mit Blättern oder Flugscharen als rotierende Einbauten versehen sind, wird das pastöse Einsatzmaterial, das über eine Pumpe eindosiert wird, gegen die beheizte Wandung geschleudert, an der die Trocknung in einer dünnen Schicht von typischerweise 1 bis 10 mm Stärke erfolgt. Im Sinne der Erfindung hat es sich als vorteilhaft erwiesen, an den Dünnschichtverdampfer einen Temperaturgradienten von 170 (Produkteinlaß) auf 20°C (Produktaustrag) anzulegen. Hierzu können beispielsweise die beiden ersten Zonen des Verdampfers auf 160°C geheizt und die letzte auf 20°C gekühlt werden. Höhere Trocknungstemperaturen haben sich im Hinblick auf die thermische Labilität bei Mitverwendung von Zuckertensiden als weiteren Einsatzstoffen als nicht vorteilhaft erwiesen. Der Dünnschichtverdampfer wird bei atmosphärischem Druck betrieben und im Gegenstrom mit Luft (Durchsatz 50 bis 150 m³/h) begast. Die Eintrittstenmperatur des Gases liegt in der Regel bei 20 bis 30, die Austrittstemperatur bei 90 bis 110°C. Die wäßrigen Monoglycerid(ether)sulfat-Pasten, die als Einsatzstoffe in Betracht kommen, können einen Feststoffgehalt im Bereich oberhalb von 20, vorzugsweise von 25 bis 75 Gew.-% aufweisen; typischerweise liegt er bei 30 bis 50 Gew.-%. Die Durchsatzmenge ist natürlich von der Größe des Trockners abhängig, liegt jedoch typischerweise bei 5 bis 15 kg/h. Es empfiehlt sich, die Pasten bei der Einspeisung auf 40 bis 60°C zu temperieren. Nach der Trocknung hat es sich weiterhin als sehr vorteilhaft erwiesen, die noch etwa 50 bis 70°C heißen Granulate auf ein Förderband, vorzugsweise eine Schwingwelle zu geben und dort rasch, d.h. innerhalb einer Verweilzeit von 20 bis 60 s, mit Umgebungsluft auf Temperaturen von etwa 30 bis 40°C abzukühlen. Zur weiteren Verbesserung der Beständigkeit gegenüber unerwünschter Wasseraufnahme kann man die Granulate auch anschließend durch Zugabe von 0,5 bis 2 Gew.-% Kieselsäure abpudern.

### Gewerbliche Anwendbarkeit

Die nach der erfindungsgemäßen Verfahren erhältlichen Granulaten können anschließend mit weiteren Inhaltsstoffen von pulverförmigen oberflächenaktiven Mitteln, wie beispielsweise Tumpulvern für Waschmittel abgemischt werden. Es ist ferner problemlos möglich, die Pulver in wäßrige Zubereitungen einzuarbeiten. Tatsächlich werden bei Verwendung der Pulver gegenüber den wäßrigen Ausgangspasten keine Unterschiede in den anwendungstechnischen Eigenschaften beobachtet. Auch gerade in Stückseifen vom Combibar- oder Syndettyp lassen sich die Granulate beispielsweise zusammen mit Fettsäuren, Fettsäuresalzen, Fettalkoholen, Stärke, Polyglycolen und dergleichen ohne weiteres einarbeiten.

### Beispiele

**Beispiel 1.** Die Herstellung der Granulate erfolgte in einem Flashdryer der VRV S.p.A., Mailand/lT. Es handelte sich hierbei um einen horizontal angeordneten Dünnschichtverdampfer (Länge 1100 mm, Innendurchmesser : 155 mm) mit 4 Wellen und 22 Blättern, deren Abstand zur Wandung 2 mm betrug. Der Trockner besaß drei separate Heiz- bzw. Kühlzonen und eine Wärmeaustauscherfläche von insgesamt 0,44 m². Der Betrieb erfolgte bei Normaldruck. Über eine Schwingpumpe wurde eine auf 50°C temperierte wäßrige Paste eines Kokosfettsäuremonoglyceridsulfat-Natriumsalzes (Plantapon® CMGS, Feststoffgehalt ca. 50 Gew.-%) mit einem Durchsatz von 11,5 kg/h in den Dünnschichtverdampfer gepumpt, dessen Heizzonen 1 und 2 auf 160°C und dessen Kühlzone 3 auf 20°C eingestellt worden waren. Die Geschwindigkeit der Rotoren betrug 24 m/s. Der Flashdryer wurde mit Luft (ca. 110 m³/h) begast; die Gasaustrittstemperatur betrug ca. 65°C. Das vorgetrocknete, noch etwa 60°C heiße Granulat wurde auf eine Schwingrinne (Länge 1 m) gegeben, mit Raumluft begast und innerhalb von 30 s auf etwa 40°C abgekühlt. Anschließend wurde es mit etwa 1 Gew.-% Kieselsäure (Sipemat® 50 S) abgepudert. Es wurde ein trockenes, rein-weißes, auch nach längerer Lagerung an der Luft rieselfähiges und nicht-klumpendes Granulat erhalten, das nach Einarbeitung in Shampoorezepturen keine Unterschiede zu einem pastösen Vergleichsprodukt zeigte. Die Kenndaten des Granulats sind in Tabelle 1 wiedergegeben.

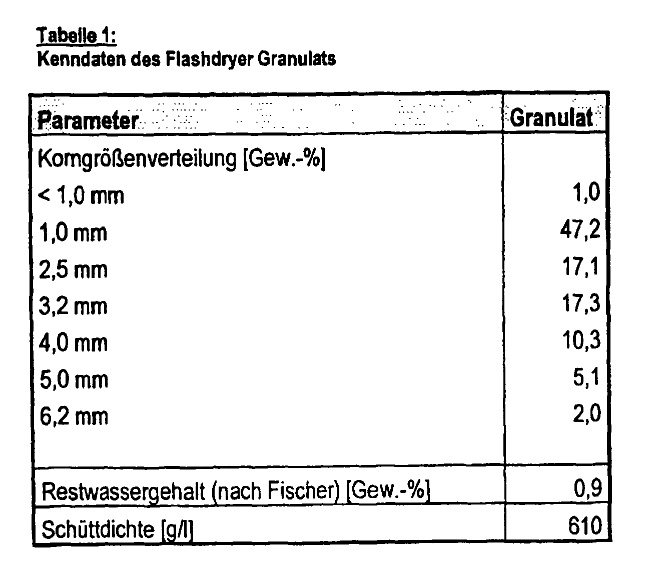

**Beispiel 2.** Beispiel 1 wurde mit einer Mischung aus 60 Gew.-% Kokosfettsäuremonoglyceridsulfat und 40 Gew.-% Kokosalkyloligoglucosid (Plantacare® APG 1200) wiederholt. Die Temperatur in den beiden Heizzonen des Flashdryers wurde auf 170°C angehoben. Es wurde ebenfalls ein rein weißes, rieselfähiges und nicht-klumpendes Granulat erhalten, das eine Schüttdichte von 600 g/l und einen Restwassergehalt von 0,8 Gew.-% aufwies.

**Beispiel 3**. Beispiel 1 wurde mit einer Mischung 60 Gew.-% Kokosfettsäuremonoglyceridsulfat und 40 Gew.-% Kokosfettalkohol (Lanette® O, Henkel KGaA) wiederholt. Die Temperatur in den beiden Heizzonen des Flashdryers wurde auf 170°C angehoben. Es wurde ebenfalls ein rein weißes, rieselfähiges und nicht-klumpendes Granulat erhalten, das eine Schüttdichte von 590 g/l und einen Restwassergehalt von 0,7 Gew.-% aufwies.

**Beispiel 4**. Beispiel 1 wurde mit einer Mischung 60 Gew.-% Kokosfettsäuremonoglyceridsulfat und 40 Gew.-% Palmkernfettsäure (Edenor PK® , Henkel KGaA) wiederholt. Die Temperatur in den beiden Heizzonen des Flashdryers wurde auf 170°C angehoben. Es wurde ebenfalls ein rein weißes, rieselfähiges und nicht-klumpendes Granulat erhalten, das eine Schüttdichte von 580 g/l und einen Restwassergehalt von 0,9 Gew.-% aufwies.

## Patentansprüche

1. Verfahren zur Herstellung von wasser- und staubfreien Aniontensidgranulaten mit hohem Schüttgewicht, bei dem man wässrige Pasten von Monoglycerid(ether)sulfaten mit einem Feststoffgehalt von mindestens 20 Gew.-% in einem horizontal angeordneten Dünnschichtverdampfer mit rotierenden Einbauten bis auf einen Restwassergehalt unterhalb von 1 Gew.-% gleichzeitig trocknet und in stückige Form bringt, wobei man an den Dünnschichtverdampfer vom Produkteingang bis zum Produktaustrag einen abnehmenden Temperaturgradienten anlegt, im Gegenstrom mit Luft begast und die Trocknung ausschließlich über die beheizte Wandung erfolgt.

2. Verfahren nach Anspruch 1, **dadurch gekennzeichnet, dass** man Monoglycerid(ether)sulfate der Formel (I) einsetzt, 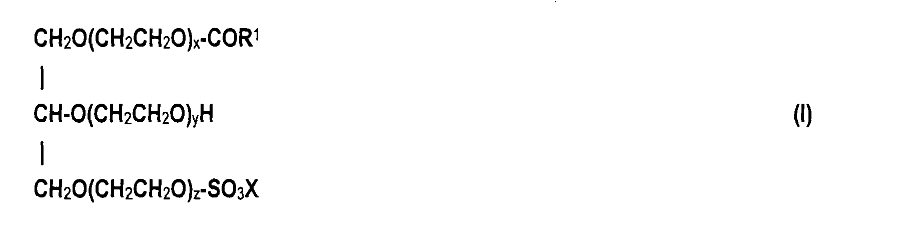 in der R¹CO für einen linearen oder verzweigten Acylrest mit 6 bis 22 Kohlenstoffatomen, x, y und z in Summe für 0 oder für Zahlen von 1 bis 30 und X für ein Alkali- oder Erdalkalimetall steht.

3. Verfahren nach den Ansprüchen 1 und 2, **dadurch gekennzeichnet, dass** man zusammen mit den Monoglycerid(ether)sulfaten Alkyl- und Alkenyloligoglykoside der Formel (II) einsetzt,
R²O-[G]ₚ (II)
in der R² für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

4. Verfahren nach den Ansprüchen 1 bis 3, **dadurch gekennzeichnet, dass** man zusammen mit den Monoglycerid(ether)sulfaten Fettsäure-N-alkylpolyhydroxy-alkylamide der Formel (III) einsetzt, 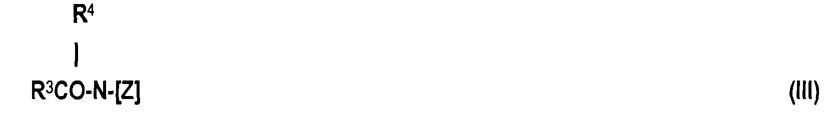 in der R³CO für einen aliphatischen Acylrest mit 6 bis 22 Kohlenstoffatomen, R⁴ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

5. Verfahren nach den Ansprüchen 1 bis 4, **dadurch gekennzeichnet, dass** man vom Produkteintrag bis zum -austrag einen Temperaturgradienten von 170 bis auf 20°C anlegt.

6. Verfahren nach den Ansprüchen 1 bis 5, **dadurch gekennzeichnet, dass** man das die noch heißen Granulate nach Ausschleusen aus dem Dünnschichtverdampfer auf einer Schwingrinne mit Umgebungsluft weiter abkühlt.

7. Verfahren nach den Ansprüchen 1 bis 6, **dadurch gekennzeichnet, dass** man die Granulate nach dem Abkühlen mit Kieselsäure abpudert.

## Revendications

1. Procédé de production de granulés d'agents tensioactifs anioniques anhydres et sans poussière ayant une densité apparente élevée dans lequel en même temps on sèche des pâtes aqueuses de monoglycéride(éther)sulfates ayant une teneur en matières solides d'au moins 20 % en poids dans un évaporateur en couche mince disposé horizontalement avec des éléments de construction tournants, jusqu'à une teneur en eau résiduelle en dessous de 1% en poids, et on les amène en forme de morceaux, procédé dans lequel on dispose dans l'évaporateur en couche mince de l'entrée du produit jusqu'à l'évacuation du produit un gradient décroissant de températures, on balaye à contre courant avec de l'air et on effectue le séchage exclusivement sur la paroi chauffée.

2. Procédé selon la revendication 1,
**caractérisé en ce qu'**
on met en oeuvre des monoglycéride(éther)sulfates de formule (I), 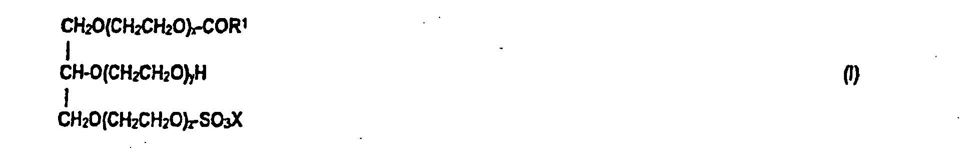 dans laquelle R¹CO représente un reste acyle linéaire ou ramifié ayant de 6 à 22 atomes de carbone, x, y et z globalement représentent O ou des nombres allant de 1 à 30, de préférence de 2 à 10 et X représente un métal alcalin ou alcalinoterreux.

3. Procédé selon les revendications 1 et 2,
**caractérisé en ce qu'**
on met en oeuvre conjointement avec les monoglycéride-(éther)sulfates, des alkyl- et alkényloligoglycosides de formule (II)
R²O-[G]ₚ (II)
dans laquelle R² représente un radical alkyle et/ou alkényle ayant de 4 à 22 atomes de carbone, G représente un reste de sucre ayant 5 ou 6 atomes de carbone et p représente des nombres allant de 1 à 10.

4. Procédé selon l'une quelconque des revendications 1 à 3,
**caractérisé en ce qu'**
on met en oeuvre conjointement aux monoglycéride-(éther)sulfates, des N-(alkylpolyhydroxyalkyl)amides de formule (III), 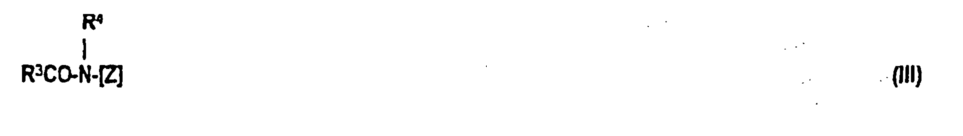 dans la quelle R³CO représente un reste acyle aliphatique ayant de 6 à 22 atomes de carbone, R⁴ représente de l'hydrogène, un radical alkyle ou hydroxyalkyle ayant de 1 à 4 atomes de carbone et [Z] représente un radical polyhydroxyalkyle linéaire ou ramifié ayant de 3 à 12 atomes de carbone et de 3 à 10 groupes hydroxyle.

5. Procédé selon l'une quelconque des revendications 1 à 4,
**caractérisé en ce qu'**
on dispose de l'introduction du produit jusqu'à l'évacuation du produit, un gradient de températures allant de 170°C à 20°C.

6. Procédé selon l'une quelconque des revendications 1 à 5,
**caractérisé en ce qu'**
on refroidit encore avec l'air environnant les granulés encore chauds après éclusage de l'évaporateur en couche mince sur un couloir vibrant.

7. Procédé selon l'une quelconque des revendications 1 à 6,
**caractérisé en ce qu'**
on saupoudre les granulés après le refroidissement avec de l'acide silicique.

## Claims

1. A process for the production of water-free and dust-free anionic surfactant granules of high bulk density, in which water-containing pastes of monoglyceride (ether)sulfates with a solids content of at least 20% by weight are simultaneously dried to a residual water content below 1% by weight and converted into particulate form in a horizontally mounted thin-layer evaporator with rotating intemals, a negative temperature gradient being applied to the thin-layer evaporator from the product entrance to the product exit, air being passed through the evaporator in countercurrent and drying taking place exclusively via the heated wall.

2. A process as claimed in claim 1, **characterized in that** monoglyceride (ether)sulfates corresponding to formula (I): 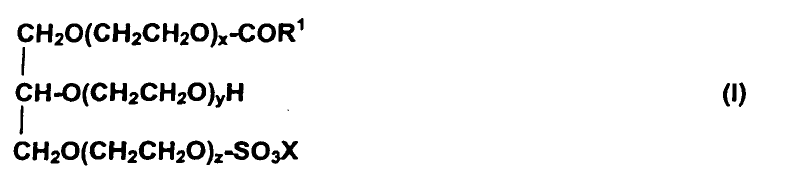 In which R¹CO is a linear or branched acyl group containing 6 to 22 carbon atoms, x, y and z together stand for 0 or for numbers of 1 to 30 and X is an alkali metal or alkaline earth metal,
are used.

3. A process as claimed in claims 1 and 2, **characterized in that** alkyl and alkenyl oligoglycosides corresponding to formula (II):
**R**_{**2**}**O-[G]**_{**p**} **(II)**
in which R² is an alkyl and/or alkenyl radical containing 4 to 22 carbon atoms, G is a sugar unit containing 5 or 6 carbon atoms and p is a number of 1 to 10,
are used together with the monoglyceride (ether) sulfates.

4. A process as claimed in claims 1 to 3, **characterized in that** fatty acid N-alkyl polyhydroxyalkylamides corresponding to formula (III): 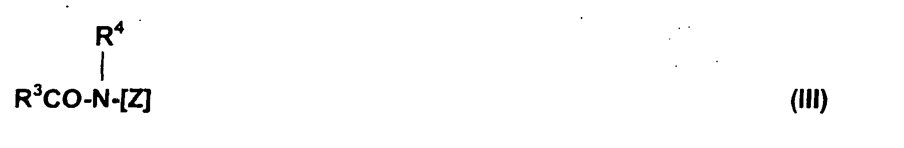 where R³CO is an aliphatic acyl radical containing 6 to 22 carbon atoms, R⁴ is hydrogen, an alkyl or hydroxyalkyl radical containing 1 to 4 carbon atoms and [Z] is a linear or branched polyhydroxyalkyl radical containing 3 to 12 carbon atoms and 3 to 10 hydroxyl groups. are used together with the monoglyceride (ether) sulfates.

5. A process as claimed in claims 1 to 4, **characterized in that** a temperature gradient from 170°C to 20°C is applied from the product entrance to product exit.

6. A process as claimed in claims 1 to 5, **characterized in that**, after removal from the thin-layer evaporator, the granules are transferred while still hot to a vibrating chute where they are further cooled with ambient air.

7. A process as claimed in claims 1 to 6, **characterized in that**, after cooling, the granules are powdered with silica.
